# EUROPEAN PATENT SPECIFICATION

(11) **EP 1 096 039 B1**
(45) Date of publication and mention of the grant of the patent: **28.12.2005**
(21) Application number: 00123350.1
(22) Date of filing: 27.10.2000
(51) Int. Cl.: C23C 22/68, C23C 8/12, C23C 8/14, A61K 6/02, A61L 27/02, A61L 27/04, A61L 27/06, A61K 6/00

(54) **Method of forming an oxide film on a metallic member and method of cementing the metallic member**
Verfahren zur Herstellung von Oxidfilmen auf einem Metallgegenstand und zur Zementierung dieses Gegenstandes
Procédé pour produire une couche d'oxyde sur une pièce métallique et pour cimenter cette pièce

(30) Priority: 29.10.1999 JP 30880999; 18.10.2000 JP 2000317987
(43) Date of publication of application: 02.05.2001
(60) Divisional application: 05017551.2
(73) Proprietor: Matsumoto Dental University, Nagano 399-0781 (JP)
(72) Inventor: Ito, Michio, Shiojiri, Nagano 399-0742 (JP)
(74) Representative: Hering, Hartmut

(56) References cited:
- EP-A- 0 396 238
- EP-A- 0 609 902
- WO-A-98/48862
- WO-A-99/11202
- DE-A- 2 546 687
- GB-A- 1 444 528
- US-A- 5 674 293
- US-A- 5 855 950
- DATABASE WPI Section Ch, Week 199503 Derwent Publications Ltd., London, GB; Class M11, AN 1995-019735 XP002186611 & JP 06 306620 A (NKK CORP), 1 November 1994 (1994-11-01)
- PATENT ABSTRACTS OF JAPAN vol. 1999, no. 06, 31 March 1999 (1999-03-31) & JP 01 136652 A (CHRISTOPHER A SARUBO), 29 May 1989 (1989-05-29)
- PATENT ABSTRACTS OF JAPAN vol. 1997, no. 10, 31 October 1997 (1997-10-31) & JP 09 170086 A (NKK CORP), 30 June 1997 (1997-06-30)

## Description

This invention relates to a method of forming an oxide film on the surface of a metallic member to be cemented to a fixing site by the use of a cementing material and to a method of cementing the metallic member.

Traditionally, in the field of dental treatment, a dental article comprising a metallic member is fixed through a cementing material to an affected part of a patient as a fixing site. Such dental article comprising the metallic member may be an orthodontic bracket, a crown or a bridge as a prosthetic material, or an inlay for conservative restoration.

As the cementing material, use is typically made of a dental cement. As a conventional cementing technique, the following methods are adopted in order to increase the adhesive strength between the metallic member and the dental cement.

In one cementing method, the surface of the metallic member is sandblasted to be polished. The metallic member with its surface sandblasted is placed on the affected part of the patient through the dental cement and then cemented by the dental cement.

In another cementing method, the metallic member to be cemented is heated in an electric furnace to form an oxide film on the surface of the metallic member. Then, the metallic member with the oxide film formed thereon is located on the affected part through the dental cement and cemented by the dental cement. In this method, the oxide film is firmly formed on the surface of the metallic member.

Before placing the metallic member on the affected part of the patient, it is necessary to partially remove the oxide film from the surface of the metallic member in an area except a cemented portion to be cemented to the dental cement and to polish the area into a mirror-finished surface.

On the other hand, an implant used in the dental field is heated in the electric furnace to be oxidized and then directly implanted in the affected part of the patient to be bonded to a living bone.

However, the metallic member requires a long polishing time to remove the oxide film as mentioned above. In addition, the metallic member may be deteriorated in quality because it is heated in the electric furnace to a high temperature to be oxidized.

It is therefore an object of this invention to provide a method of forming an oxide film on a metallic member, which is capable of avoiding the deterioration in quality of the metallic member as a result of heat treatment to form the oxide film and which is capable of easily forming the oxide film in a predetermined portion of the metallic member.

It is another object of this invention to provide a method of cementing a metallic member, which is capable of avoiding the deterioration in quality of the metallic member as a result of heat treatment to form the oxide film, which does not require a long polishing time, and which is capable of improving reliability and strength of cementing.
Fig. 1 is a graph showing an adhesive strength according to each of second and third embodiments of this invention;
Fig. 2 is a graph showing an adhesive strength according to a fourth embodiment of this invention;
Fig. 3 is a graph showing a bonding strength in case where the second embodiment of this invention is applied to an implant; and
Fig. 4 is a graph similar to Fig. 7 in case where the first embodiment of this invention is applied to an implant.

Next, description will be made about a method of forming an oxide film on a metallic member and a method of cementing a metallic member according of this invention.

In the oxide film forming method of the second embodiment, the oxide film is formed on the surface of a predetermined portion of the metallic member by the use of ozone (O₃).

In the cementing method of the second embodiment, the oxide film is at first formed on the metallic member to be cemented. Specifically, the oxide film is formed by the use of ozone. Thereafter, the metallic member with the oxide film formed thereon is cemented through the cementing material to a fixing site of a patient as an affected part.

In an experimental test in the second embodiment, ozone was generated by an ozone generator to be filled in a box. Metal plates of pure titanium, a titanium alloy, stainless steel, a nickel-chromium alloy, a cobalt-chromium alloy, a gold-silver-palladium alloy, a gold alloy, and a silver alloy were placed in the box so that the surface of each of the metal plates was oxidized. After oxidization, the metal plates are cemented to each other through a resin-based adhesive as the cementing material.

As the resin-based adhesive, use may be made of a mixture of polymethyl methacrylate (PMMA) powder and a methylmethacrylate solution, a mixture of PMMA and 2,2-bis[4-(3-methacryloxy-2-hydroxypropoxy)] phenyl propane, and a mixture of PMMA and triethylene glycol dimethacrylate.

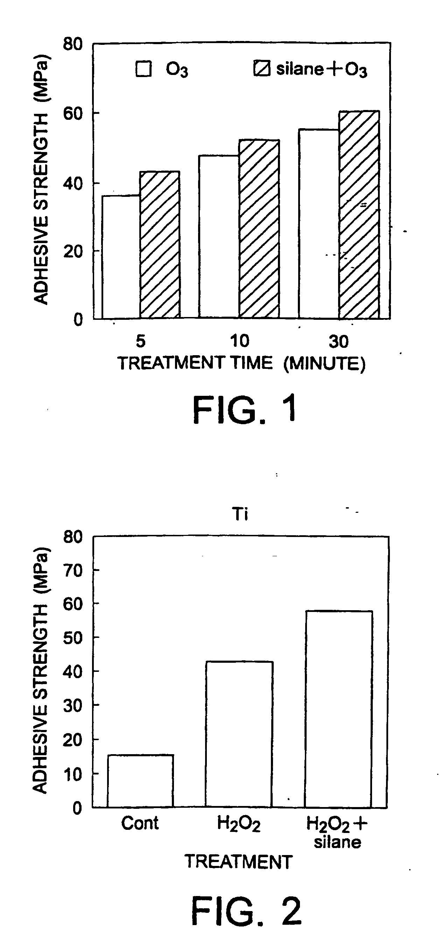

Referring to Fig. 1, the adhesive strength (MPa) achieved via the oxide film treatment by ozone and adhesion by the resin-based adhesive is illustrated by white bars in the figure. The measurement of the adhesive strength was performed in the following manner.

At first, a plurality of pairs of metal plates of a 10mm square were prepared as metallic members. The metal plate pairs as samples were grouped into first through fourth groups. An oxide film was formed on each metal plate in the manner which will be described below. Then, the metal plates in each sample were cemented to each other by the use of a cementing material. Thereafter, one of the metal plates in each sample was given a load in a direction parallel to a plate plane to measure the adhesive strength (MPa: megapascal) against shearing force. In addition to the samples, for each of the first through the fourth groups, preparation was also made of a control (Cont) in which no oxide film was formed on the metal plates cemented to each other.

When the treatment time by ozone was 5 minutes, the adhesive strength was about 38MPa. As the treatment time was prolonged to 10 minutes and 30 minutes, the adhesive strength was gradually increased to about 47MPa and about 55MPa, respectively.

Next, description will be made of a method of forming an oxide film on a metallic member and a method of cementing a metallic member according to a third embodiment of this invention.

In the oxide film forming method of the third embodiment, the oxide film is formed on the surface of a predetermined portion of the metallic member by the use of ozone in the manner similar to that mentioned in conjunction with the second embodiment. Thereafter, silane (namely, γ-methacryloxy propyl triethoxy silane) is applied as a coupling agent on the oxidized surface of the metallic member, i.e., on the oxide film.

In the cementing method of the third embodiment, the oxide film is at first formed on the surface of the predetermined portion of the metallic member to be cemented. Specifically, the oxide film is formed by the use of ozone. Thereafter, the silane coupling agent is applied on the oxidized surface, i.e., on the oxide film. Then, the metallic member is cemented through a cementing material to a fixing site of a patient as an affected part.

In an experimental test in the third embodiment, the oxide film was formed on the metal plate by the use of ozone, in the manner similar to that mentioned in conjunction with the second embodiment. Then, the silane was applied as the coupling agent on the oxidized surface, i.e., on the oxide film. Thereafter, the metal plates with the oxide films formed thereon were cemented to each other by the use of the cementing material.

In the manner similar to that earlier mentioned the adhesive strength was measured. The result of measurement is depicted by hatched bars in Fig. 1. When the treatment time was 5 minutes, the adhesive strength was about 42MPa. As the treatment time was prolonged to 10 minutes and 30 minutes, the adhesive strength was gradually increased to about 51 MPa and about 60MPa, respectively. Thus, the adhesive strength was slightly greater than the oxide film treatment by ozone alone.

Next, description will be made of a method of forming an oxide film on a metallic member and a method of cementing a metallic member according to a fourth embodiment of this invention.

In the oxide film forming method of the fourth embodiment, the oxide film is formed on the surface of a metallic member by treating the surface with a 34% hydrogen peroxide solution and thereafter treating the oxidized surface with the silane coupling agent.

In the cementing method of the fourth embodiment, the oxide film is at first formed on the surface of the metallic member by treating the surface with the 34% hydrogen peroxide solution and thereafter treating the oxidized surface with the silane coupling agent. Then, the metallic member is cemented through a resin-based adhesive to the fixing site of a patient as an affected part.

Referring to Fig. 2, the adhesive strength was measured in an experimental test of the fourth embodiment. Specifically, a titanium plate as the metallic member was treated by a 34% hydrogen peroxide solution to form the oxide film, treated by a silane coupling agent, and cemented by a resin-based adhesive to another titanium plate similarly treated. Then, the adhesive strength was measured in the manner similar to that mentioned earlier. Preparation was also made of the control in which titanium plates without the treatment by the 34% hydrogen peroxide solution, i.e., without the oxide films were cemented to each other by the resin-based adhesive.

As illustrated in Fig. 2, the adhesive strength was 16MPa in the control and was 42MPa in the treatment by the 34% hydrogen peroxide alone. By a combination of the treatment by the hydrogen peroxide treatment and the treatment by the silane coupling agent, a most excellent adhesive strength of 58MPa was achieved.

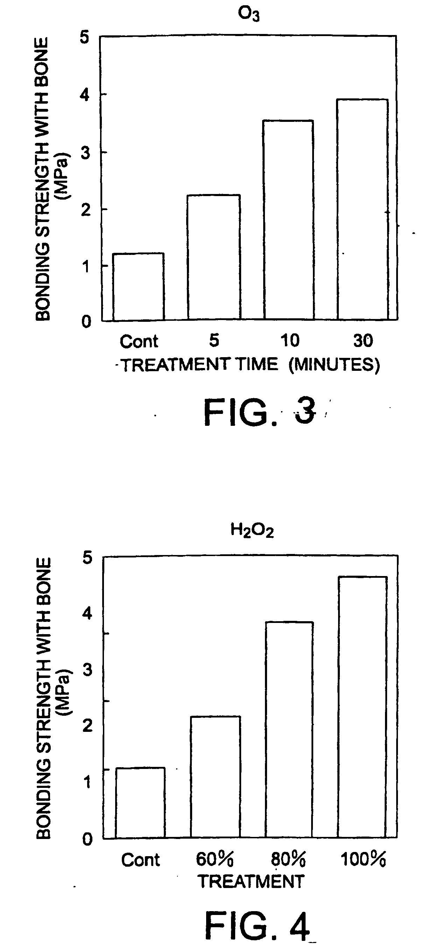

Referring to Fig. 3, a titanium implant was treated by ozone to form an oxide film on the surface of the implant in the manner similar to that mentioned in conjunction with the second embodiment. The implant with the oxide film formed thereon was implanted to a tibia or shinbone of a rabbit. After lapse of eight weeks, the implant was subjected to measurement of bonding strength with the bone by means of the pull-out test. The result of measurement is shown in Fig. 3. Herein, preparation was also made of the control in which the implant without the ozone treatment, i.e., without the oxide film was implanted to the tibia of the rabbit.

As seen from Fig. 3, the bonding strength (MPa) between the bone and the ozone-treated implant was 2.2MPa when the ozone treatment time was five minutes and, as the treatment time was prolonged to 10 minutes and 30 minutes, the bonding strength was gradually increased. This proves that the wettability of a bone tissue is improved by the presence of the oxide film.

Referring to Fig. 4, the titanium implant was treated by the hydrogen peroxide solution in three different concentrations to form the oxide film on the surface of the implant in the manner similar to that mentioned in conjunction with the first embodiment. Specifically, the 34% hydrogen peroxide solution as a 100% starting solution was diluted to 60%, diluted to 80%, and not diluted. The titanium implant was dipped in the hydrogen peroxide solutions for several minutes. Thereafter, the implant was implanted to the tibia of the rabbit and, after lapse of eight weeks, the bonding strength with the bone was measured by the pull-out test.

The result of measurement is shown in Fig. 4. Preparation was also made of the control in which the implant without the hydrogen peroxide treatment, i.e., without the oxide film was implanted to the tibia of the rabbit.

From the result of measurement, the titanium implant treated by the hydrogen peroxide solution required a greater drawing force than that required in the titanium implant without such treatment, i.e., without the oxide film. Presumably, the bonding strength between the bone and the titanium implant was improved by the treatment with the hydrogen peroxide treatment. Thus, it has been revealed that the wettability of the bone tissue is improved by the oxide film formed by the treatment with the hydrogen peroxide solution.

As described above, according to the oxide film forming method of this invention, the oxide film similar to that obtained by the conventional heat treatment is formed on the surface of the metallic member within several minutes by the use of hydrogen peroxide, ozone, and the silane coupling agent, alone or in combination.

Thus, the metallic member need not be heated and oxidized in the electric furnace and oxidation can reliably be carried out in a short time.

According to the cementing method of this invention, the oxide film is formed on the surface of the metallic member by the use of ozone or in combination with the silane coupling agen and the metallic member is cemented to the affected part through the cementing material.

As described above, the process from the oxidation to the cementing can be quickly and reliably carried out without requiring the heat treatment and the oxidation in the electric furnace.

According to the oxide film forming method and the cementing method of this invention, it is possible to avoid the deterioration in quality of the metallic member due to the high-temperature heat treatment and to save the time for polishing the surface of the metallic member.

## Claims

1. A method of forming an oxide film on a metallic member included in a dental article, said method being for forming said oxide film on the surface of a predetermined portion of said metallic member, comprising the step of oxidizing the surface of said predetermined portion by the use of ozone to form said oxide film, proviso the method does not include the case of treatment on human or animal body.

2. A method as claimed in claim 1, further comprising the step of:
applying silane on said oxide film as a coupling agent.

3. A method as claimed in claim 1 or 2, wherein said-metallic member is formed by a material selected from a group consisting of a cobalt-chromium alloy, a nickel-chromium alloy, stainless steel,' pure titanium, a titanium alloy, a platinum gold alloy, a gold-silver-palladium alloy, a silver alloy, and a gold alloy.

4. A method as claimed in any of claims 1 to 3, wherein said metallic member is an implant.

5. A method of fastening a metallic member of a dental article to a fixing part included in an affected part of a prosthetic material or a material for conservative restoration comprising the steps of:
oxidizing the surface of a predetermined portion of said metallic member by ozone to form said oxide film;
applying a cementing material on the oxide film; and
cementing said predetermined portion to said fixing part through said cementing material to fix the metallic member to the fixing part, proviso the method does not include the case of treatment of human or animal body.

6. A method as claimed in claim 5, further comprising the step of:
applying silane as a coupling agent to the oxide film before the cementing step.

7. A method as claimed in claim 5 or 6, wherein said metallic member is formed by a material selected from a group consisting of a cobalt-chromium alloy, a nickel-chromium alloy, stainless steel, pure titanium, a titanium alloy, a platinum gold alloy, a gold-silver-palladium alloy, a silver alloy, and a gold alloy, said cementing material being a dental cement.

8. A method as claimed in claim 5 to 7, wherein said metallic member is selected from a group including a cobalt-chromium alloy, a nickel-chromium alloy, stainless steel, pure titanium, a titanium alloy, a platinum gold alloy, a gold-silver-palladium alloy, a silver alloy, and at gold alloy, said cementing material being a resin-based adhesive.

## Patentansprüche

1. Verfahren zur Herstellung eines Oxidfilms auf einem Metallgegenstand, der in einem Dentalprodukt enthalten ist, dieses Verfahren dient der Herstellung eines Oxidfilms auf der Oberfläche eines vorherbestimmten Teils des Metallgegenstandes, umfassend den Schritt
Oxidation der Oberfläche des vorherbestimmten Teils durch die Verwendung von Ozon, um den Oxidfilm zu bilden,
vorausgesetzt, daß das Verfahren nicht den Fall der Behandlung des menschlichen oder tierischen Körpers einschließt.

2. Verfahren nach Anspruch 1, weiterhin umfassend den Schritt Anwendung von Silan auf den Oxidfilm als Kupplungsmittel.

3. Verfahren nach Anspruch 1 oder 2, wobei der Metallgegenstand gebildet wird, aus einem Material ausgewählt aus der Gruppe, bestehend aus Kobalt-Chrom-Legierung, Nickel-Chrom-Legierung, rostfreier Stahl, reines Titan, Titanlegierung, Platin-Gold-Legierung, Gold-Silber-Palladium-Legierung, Silberlegierung und Goldlegierung.

4. Verfahren nach einem der Ansprüche 1 bis 3, bei dem der Metallgegenstand ein Implantat ist.

5. Verfahren zur Befestigung eines Metallgegenstandes eines Dentalartikel an einem Fixierteil, das in einem betroffenen Teil eines prothetischen Material oder einem Material für die konservative Wiederherstellung vorhanden ist, umfassend die Schritte
Oxidation der Oberfläche eines vorherbestimmten Teils eines Metallgegenstandes mit Ozon, um einen Ozonfilm herzustellen:
Anwendung eine Bindemittels auf dem Ozonfilm; und
Verkleben des vorherbestimmten Teils mit dem Fixierteil durch das Bindemittel, um den Metallgegenstand an dem Fixierteil zu fixieren, vorausgesetzt, daß das Verfahren nicht den Fall der Behandlung des menschlichen oder tierischen Körpers einschließt.

6. Verfahren nach Anspruch 5, weiterhin umfassend den Schritt Anwendung von Silan als Kupplungsmittel auf den Oxidfilm bevor der Klebeschritt ausgeführt wird.

7. Verfahren nach Anspruch 5 oder 6, bei dem der Metallgegenstand gebildet wird, aus einem Material ausgewählt aus der Gruppe, bestehend aus Kobalt-Chrom-Legierung, Nickel-Chrom-Legierung, rostfreier Stahl, reines Titan, Titanlegierung, Platin-Gold-Legierung, Gold-Silber-Palladium-Legierung, Silberlegierung und Goldlegierung, das Bindemittel ein Dentalkleber ist.

8. Verfahren nach einem der Ansprüche 5 bis 7, bei dem Metallgegenstand gebildet wird, aus einem Material ausgewählt aus der Gruppe, bestehend aus Kobalt-Chrom-Legierung, Nickel-Chrom-Legierung, rostfreier Stahl, reines Titan, Titanlegierung, Platin-Gold-Legierung, Gold-Silber-Palladium-Legierung, Silberlegierung und Goldlegierung, das Bindemittel ein Klebstoff auf Harzbasis ist.

## Revendications

1. Procédé pour former un film d'oxyde sur un élément métallique inclus dans un article dentaire, ledit procédé étant pour former ledit film d'oxyde sur la surface d'une portion prédéterminée dudit élément métallique, comprenant l'étape consistant à :
oxyder la surface de ladite portion prédéterminée au moyen d'ozone pour former ledit film d'oxyde, à condition que le procédé ne comprenne pas le cas du traitement sur le corps d'un être humain ou d'un animal.

2. Procédé selon la revendication 1, comprenant en outre l'étape consistant à :
appliquer du silane sur ledit film d'oxyde comme agent adhésif.

3. Procédé selon la revendication 1 ou 2, dans lequel ledit élément métallique est formé par un matériau sélectionné parmi un groupe composé d'un alliage cobalt-chrome, d'un alliage nickel-chrome, d'acier inoxydable, de titane pur, d'un alliage de titane, d'un alliage d'or platiné, d'un alliage or-argent-palladium, d'un alliage d'argent et d'un alliage d'or.

4. Procédé selon l'une quelconque des revendications 1 à 3, dans lequel ledit élément métallique est un implant.

5. Procédé de fixation d'un élément métallique d'un article dentaire sur une pièce de fixation incluse dans une partie atteinte d'un matériau de prothèse ou d'un matériau de restauration conservatrice, comprenant les étapes consistant à :
oxyder la surface d'une portion prédéterminée dudit élément métallique par de l'ozone pour former ledit film d'oxyde ;
appliquer un matériau de cimentation sur le film d'oxyde ; et
cimenter ladite portion prédéterminée sur ladite pièce de fixation par l'intermédiaire dudit matériau de cimentation pour fixer l'élément métallique sur la pièce de fixation, à condition que le procédé ne comprenne pas le cas du traitement sur le corps d'un être humain ou d'un animal.

6. Procédé selon la revendication 5, comprenant en outre l'étape consistant à :
appliquer du silane en tant qu'agent adhésif sur le film d'oxyde avant l'étape de cimentation.

7. Procédé selon la revendication 5 ou 6, dans lequel ledit élément métallique est formé par un matériau sélectionné parmi un groupe composé d'un alliage cobalt-chrome, d'un alliage nickel-chrome, d'acier inoxydable, de titane pur, d'un alliage de titane, d'un alliage d'or platiné, d'un alliage or-argent-palladium, d'un alliage d'argent et d'un alliage d'or, ledit matériau de cimentation étant un ciment dentaire.

8. Procédé selon l'une des revendications 5 à 7, dans lequel ledit élément métallique est sélectionné parmi un groupe comprenant un alliage cobalt-chrome, un alliage nickel-chrome, de l'acier inoxydable, du titane pur, un alliage de titane, un alliage d'or platiné, un alliage or-argent-palladium, un alliage d'argent et un alliage d'or, ledit matériau de cimentation étant une colle à base de résine.
